# EUROPEAN PATENT SPECIFICATION

(11) **EP 1 600 151 B1**
(45) Date of publication and mention of the grant of the patent: **13.08.2008**
(21) Application number: 04010180.0
(22) Date of filing: 29.04.2004
(51) Int. Cl.: A61K 8/25, A61Q 13/00, C11D 3/50, C11D 3/12

(54) **Perfume particles and a process for preparing the same**
Parfumteilchen und das Verfahren zu deren Herstellung
Particules de parfum et procédé pour leur preparation.

(43) Date of publication of application: 30.11.2005
(73) Proprietor: KAO CORPORATION, Chuo-Ku Tokyo (JP)
(72) Inventor: Wollmann, Gerhard, Dr., 40723 Hilden (DE); Uhl-Venning, Susanne, 6524 CP Nimwegen (NL)
(74) Representative: HOFFMANN EITLE

(56) References cited:
- EP-A- 0 820 762
- GB-A- 2 066 839
- US-A- 5 876 755
- US-B1- 6 608 017

## Description

### Field of the invention

The present invention relates to perfume particles suitable to be used in powder detergents and cleansing agents, and, in particular, in household products, cosmetics, personal care products and sanitary products, and to a method for preparing the same.

### Background of the invention

The addition of perfumes to detergents and cleansing agents is aimed at masking the malodour of components of these compositions during aqueous cleaning, washing or drying procedures.

Perfumes used in detergent compositions should provide the washed textile with a pleasant fragrance in the humid and also in the dried state. Furthermore, the perfume should be chosen such that the fragrance of the textile in the dried state remains constant over a long time period.

In contrast thereto, when choosing a perfume for a cleansing agent, the perfume should not adhere to the cleaned materials because they should not take on the fragrance of the perfume. Such a perfume should not adsorb to materials such as glass, ceramic, porcelain, PVC, polyethylene, polypropylene, melamine, styrene acrylonitrile, steel, stainless steel and varnishes of all sorts.

Perfumes can be added either directly to the detergent or the cleansing agent or they can be added in form of previously produced perfume particles comprising perfume dispersed within a carrier material.

The advantage of adding previously produced perfume particles instead of the liquid perfume to the detergent or the cleansing agent is a higher perfume yield during the washing step and an often prolonged effect through controlled release of the perfume. Furthermore, the addition of perfume particles to powdery compositions does not require a liquid handling step.

EP 0 820 762 A1 relates to perfume compositions comprising silica particles and perfume. A method of manufacture of perfumed detergents by adding perfume to the ingredients of the detergents is described in GB 2 066 839.

Heavily perfumed particles are disclosed in US 4,209,417, consisting essentially of from 30% to 70% water-insoluble perfume, from 25% to 65% of a water-soluble polymer and an emulsifier. Cellulose and poly(acrylic acid) derivatives are mentioned as part of a list of suitable water-soluble polymers. The perfume particles described by means of examples in US 4,209,417 consist of polyvinyl alcohol, perfume and ditallow dimethyl ammonium chloride. The preparation process described in the US document comprises forming an aqueous dispersion of the perfume, the water-soluble polymer and the emulsifier, casting the dispersion on a surface for drying, and pulverizing the dried film.

A shaped body containing odoriferous substances is described in WO97/08289. The document also mentions the preparation of premixed compositions of perfume, carrier material and e.g. filling materials: The premixed compositions described by examples in WO97/08289 comprise Portil A (water glass) and perfume besides further components. Other compounds suitable as perfume carriers according to this document are for example starch and silica (such as Sipernat). The shaped bodies (e.g. tablets or spheres) of WO97/08289 are obtained by irradiation of mixtures comprising the carrier material, perfume and/or filling materials and they can be added to the washing machine during the washing step in order to apply the contained perfume onto the washed textile.

Perfume particles comprising perfume dispersed in a water-insoluble non-polymeric carrier material encapsulated by a protective shell by coating with a friable coating material such as an aminoplast polymer is described in WO92/18601.

Starch encapsulated oil particles are described in US 6,608,017 Bl. US 5,876,755 relates to a composition comprising a substance encapsulated within a water-sensitive matrix.

A coating or encapsulation process for the production of solid particles is described in EP 0 382 464. This process comprises a first step of forming a melt of a coating material with solid particles or liquid droplets as a dispersed phase in the melt, and a second step of destabilizing the melt by the addition of solid particles causing the melt to crumble to a particulate product. The perfume particles mentioned in EP 0 382 464 consist of 35% silica carrying 40% of adsorbed perfume as the dispersed phase, 20% polycaprolactone as coating material and 5% Aerosil 380 as crumbling agent.

US 4,954,285 refers to perfume particles formed by absorbing a perfume composition onto silica particles. A perfumed detergent composition is prepared by mixing the preformed perfume/silica gel composition with a granular detergent composition.

Perfume particles consisting of porous hydrated silicon dioxide and perfume are mentioned in GB 2,066,839.

The multitude of publications in the sector of perfumed powder detergents and cleansing agents for their use in aqueous cleaning and washing procedures shows that there is a high interest for inexpensive perfume particles, which are biodegradable.

### Summary of the invention

The present invention relates to flowable perfume particles. The perfume particles of the present invention comprise a perfume, a water-soluble or in water dispersible carrier material, the carrier material being selected from builders and anti-redeposition agents, and a water-insoluble silica in an amount of 1-5 wt.% based on the total weight of the carrier material, the perfume and the powdering agent, the perfume particles being obtainable by spraying the perfume onto the carrier material and subsequently adding the powdering agent during mixing.

Furthermore, a process is provided for producing the perfume particles of the present invention. The process comprises first spraying the perfume onto the carrier material, which is supplied in the form of particles, during agitation of the carrier material and subsequently adding the powdering agent during mixing. This process leads to the wetting of the carrier material by the perfume, and the adhesion of the powdering agent to the perfume on the carrier surface and to the carrier.

The perfume particles of the present invention are suitable for their use in powder detergents and cleansing agents, in particular household products, cosmetics, personal care products and sanitary products.

### Detailed description of the invention

The following description of preferred embodiments is aimed at the illustration of the present invention.

### Perfume particles

Perfumes, carrier materials, powdering agents and further optional ingredients suitable for their use in the perfume particles of the present invention are exemplified in the following.

### Examples for suitable perfumes

A perfume means any odoriferous substance or any material able to mask malodours from detergents and cleansing agents. In general, perfumes are characterized by a vapour pressure greater than atmospheric pressure at ambient temperature. A wide variety of chemical substances is known for their use as perfumes, such as natural products, alcohols, ketones, aldehydes, nitriles, oxides, alkenes, ketenes, acetates, esters, lactones and ethers. A perfume may be represented by a single substance or consist of mixtures of only a few or more than 50 different chemicals.

Preferably used perfumes may be selected either singly or as a mixture from the group consisting of
Natural products such as camphor, coumarin, Galaxolide®, heliotropin, musk-amberette, musk-ketone, musk-tibetene, musk-xylol, ethyl-vanillin, vanillin, evernia furfuraceae (tree moss) absolute, basil oil, bergamot oil, mandarin oil, patchouli oil, petitgrain oil, absinth oil, myrrh oil, sandalwood oil, guaiac wood oil, cabreuva,
Alcohols such as alpha-terpineol, anethole, aurantiol, benzyl alcohol, cinnamic alcohol, citronellol, cyclohexyl ethanol, dihydrolinalool, dihydromyrcenol, dimethyl phenyl carbinol, eucalyptol, eugenol, farnesol, fenchol, floralol, geraniol, isoborneol, isocyclogeranol, isoeugenol, linalool, Muguetanol, myrcenol, nerol, nerolidol, nonadylalcohol, patchouly alcohol, phenyl-ethyl-alcohol, phenyl-propanol, phenyl-hexanol, rhodinol, sandela, Sandalore®, terpineol,
Naphtalenes such as acetyl hexamethyl tetrahydro-naphthalene,
Aldehydes such as anisaldehyde, benzaldehyde, benzylformate, methyl-nonyl-acetaldehyde, citral, neral, citronellal, decanal, geranial, Helional®, alpha-hexylcinnamaldehyde, hydroxycitronellal, Lilial®, alpha-amylcinnamaldehyde, veratraldehyde,
Ketones such as benzophenone, menthone, alpha-methylionone, gamma-methyl-ionone,
Alkenes such as camphene, alpha-cedrene, beta-cedrene, d-limonene, paracymene, myrcene, alpha-pinene, beta-pinene, gamma-terpinene, C₁₅H₂₄ sesquiterpenes, carophyllene, betaselinene,
Nitriles such as geranyl-nitrile,
Oxides such as linalooloxide,
Acetates such as allyl phenoxyacetate, benzylacetate, dimethyl benzyl carbinyl acetate, isobornyl acetate,' cinnamylpropionate, citronellyl acetate, cyclohexylsalicylate, decyl acetate, ethyl acetate, Evernyl®, geranyl acetate, cis-3-hexenyl acetate, hexenyl isobutyrat, isobornyl isobutyrate, linalyl acetate, methyl-phenyl-carbinyl-acetate, laevo-menthyl acetate, methyl dihydrojasmonate, myrcenyl acetate, neryl-acetate, nonyl-acetate, terpinyl-acetate, butyl-cyclohexyl acetate, cis-3-hexenyl-salicylate, hexyl-salicylate, trichloromethyl phenyl carbinyl acetate, tri-ethyl-citrate, phenyl-ethyl-phenylacetate, vetiveryl acetate,
Esters such as benzyl salicylate, linalyl-propionate, hexahydro-methano-indenyl-propionate, methyl anthranilate, iso-amyl-salicylate,
Lactones such as gamma-undecalacton, ethylene brassylate.
Ethers such as Herbavert, Ambroxan,
as well as various further components often used in the perfume industry such as indole, p-menthane-8-thiol-3-on and methyleugenol.

According to a preferred embodiment of the present invention, the amount of perfume is in the range of 10 to 35 wt.% of the total weight of the carrier material, the perfume and the powdering agent.

### Examples for suitable carrier materials for perfume particles

The water-soluble or in water easily-dispersible carrier material comprised in the perfume particles of the present invention is selected from builders and anti-redeposition agents (graying inhibitors), which are generally included in non-perfumed powdery detergents and/or cleansing agents. A non-noxious component is preferably chosen as the carrier material, which does not leave traces on cleaned surfaces and which is either biologically degradable or inert.

Builders and anti-redeposition agents (graying inhibitors) are known from the state of the art.

Builder is a term well-known to the person skilled in the art and refers to one of the key components of detergents, eliminating calcium and magnesium ions present in hard water and preventing the deposition of calcium and magnesium salts on the cleaned surfaces and in the washing- or dishwashing-machines.

According to a preferred embodiment of the present invention, water-soluble poly(meth)acrylic acid and salts thereof are used as carrier materials.

Especially preferred are (maleic acid/acrylic acid) copolymers and salts thereof.

According to a further more preferred embodiment of the present invention, maleic acid/olefin copolymers and salts thereof are used as the carrier.

According to a specific embodiment of the present invention, the carrier material is represented by a water-soluble sodium salt of the copolymerisate of maleic-acid/acrylic acid with a remaining humidity of not more than 8 wt.%.

Anti-redeposition agents (graying inhibitors) prevent the redeposition of dirt from the suds on cleaned surfaces by acting as an adsorber and dispersant. Anti-redeposition agents comprise fibre like adsorbers such as cellulose, cellulose derivatives, starch, hydrolysates of starch, sucrose, polysaccharide acetate, and silicates.

According to a preferred embodiment of the present invention, the carrier material is represented by an alkaline silicate such as water glass. Water glass is also known as corrosion inhibitor.

According to a specific embodiment of the present invention, the carrier material is represented by an alkaline, hydrated sodium water glass with a remaining humidity of 18 to 20 wt.%.

The powdering agent of the present invention adheres to the perfume on the carrier surface and to the carrier. It exhibits a spacer-like function and prevents the perfume particles from sticking together during storage and/or under pressure.

The powdering agent used according to the present invention is water-insoluble silica.

The powdering agent is present in an amount of 1-5 wt.% based on the total weight of the perfume, the carrier material and the powdering agent.

In particular, the commercially available silicas from Degussa Sipernat 22 (average particle size 100 µm), Sipernat 22 S (average particle size 7.0 µm), Sipernat 50 (average particle size 50 µm), Sipernat 320 (average particle size 15 µm), Sipernat 2200 (average particle size 300 µm) and Aerosil 200 are suitable.

Optional further components, which may be comprised in the perfume particles of the present invention are preferably selected from the group of ingredients commonly used in detergents and cleansing agents such as surfactants and auxiliary agents, e.g. dyes or anti-oxidants. A list of suitable ingredients of detergents are described in US 6,025,319 and US 6,616,705. The chemical lexicon Ullmann's encyclopedia of Industrial Chemistry' (5th edition, published by VCH, Germany, 1996) provides an overview of ingredients contained in detergents and cleansing agents.

### Preparation of perfume particles

The present inventors established a method for the production of non-sticky, free-flowable perfume particles being resistant to storage meaning that no significant alteration of the properties, such as the flowability and the fragrance, of the perfume particles occurs during storage. The water-soluble or in water dispersible carrier material is supplied in the form of particles. In general, separation of the particles of the carrier material can be achieved by agitating the solid in a commercially available solid mixer.

Examples for commercially available solid mixers for their use in the preparation of perfume particles are mixers suitable for dispersing liquids in a solid material, such as horizontal mixers, which are offered by companies such as Lödige, Papenmeier, Drais Gericke and Hosokawa-Micron for discontinuous as well as for continuous operation; vertical mixers for discontinuous as well as for continuous operation, which are commercially available for example from the companies Papenmeier, MIT, and Hosokawa-Schurgi; and conical mixers, which are available from Hosokawa-Micron and Nauta.

The perfume is atomised by the means of a commercially available perfume sprayer and sprayed onto the carrier material during agitation. The amount of the perfume is chosen such that the resulting wetted carrier material remains flowable. Then, water-insoluble silica is added as powdering agent during mixing, adhering to the perfume on the carrier surface and to the carrier.

Free-flowable perfume particles are obtained, which are dust-free and show a constant fragrance over a long time period. The perfume particles are resistant to storage in a temperature range of -10 to 30°C. The fragrance of the perfume particles after longer storage is consistent with the fragrance at the time when the perfume particles are first packed. Furthermore, the fragrance of detergents or cleansing agent prepared by the addition of perfume particles of the present invention during their use in an aqueous cleaning or washing procedure is similar or identical to the fragrance of a formulation, which has been prepared by conventionally adding liquid perfume or by other established processes.

Perfume particles having a particle size similar to that of the non-perfumed powder detergent or cleansing formulation particles can be obtained by the above described processes. Based on the similar particle sizes, separation of the perfume particles from the formulation particles during transportation and storage is avoided.

Mixing of perfume particles with the final formulation can be executed for example by means of the commercially available solid mixers described above. The perfume particles can be added shortly before packaging to the non-perfumed formulation for the customer specific design of the fragrance of the final formulation.

### Water-soluble or dispersible in water

The carrier material used in the preparation of the perfume particles is defined as water-soluble if at least 10 g of the perfume particles completely dissolve in 45 1 of water or as dispersible if at least 10 g of the perfume particles are dispersed in water during agitation 5 minutes after the addition of 45 1 of water, the term 'dispersed' indicating that no visible agglomerates are formed and no sedimentation occurs. The water-solubility or the dispersion is measured at room temperature.

### Testing the flowability of the perfume particles

The perfume particles are classified as flowable if the flow rate of 100 ml of the particles, measured according to JIS K 3362, is 10 s or less.

### Examples

The present invention will be explained concretely by the following Examples.

### Perfume particles for detergent formulations

Any perfume composition suitable to be used in a detergent formulation, in the following called 'detergent perfume', can be employed in the preparation of the perfume particles described below.

### Example 1

16 g of a water-soluble sodium salt of the copolymer of maleic acid/acrylic acid with a weight molecular weight of 70,000 kg/kmol (trade name Sokalan CP 5, commercially available from BASF) was provided in a Petri dish. 4 g of the perfume oil 'detergent perfume' were sprayed by the means of an atomizer and dispersed onto the polymer particles under agitation.

Subsequently, 4 wt.% of water-insoluble silica (trade name Sipernat 50, commercially available from Degussa), based on the total weight of the polymer, the detergent perfume and the silica, were added as powdering agent during mixing to yield flowable, non-sticky and non-adhering perfume particles.

### Example 2

32 g of water-soluble sodium water glass (trade name Portil A, CAS 1344-09-8, commercially available from Cognis, bulk density 600-800 kg/m³) with a residual humidity of 18-20% by weight were provided in a Petri dish. 8 g of perfume oil 'detergent perfume' were sprayed by the means of an atomizer and dispersed onto the water glass during agitation.

Subsequently, 4.5 wt.% of water-insoluble silica (trade name Sipernat 22, commercially available from Degussa), based on the total weight of the water glass, the perfume and the silica, were added as powdering agent during mixing. Non-sticky, flowable perfume particles on Portil A were obtained.

### Preparation of detergent formulations

The perfume particles produced as described above in examples 1 and 2 were mixed with non-perfumed detergent formulations to obtain compositions comprising a perfume concentration of 0.1 wt.%. As comparative example, perfume oil 'detergent perfume' was added to the non-perfumed laundry detergent, also yielding in a perfume concentration of 0.1 wt.% based on the total weight of the final formulation. The resulting formulations were tested for their own fragrance and for the fragrance of the washed textiles.

### Example 3

300 g of a perfume-free laundry detergent (trade name Persil perfume-free, commercially available from Henkel) were provided in a 2 1 sample container.

1.5 g of perfume particles on Sokalan prepared according to Example 1 were added to the above described detergent formulation so that a perfume concentration of 0.1 wt.% based on the total weight of the final perfumed washing agent formulation was obtained and the mixture was shaken vigorously.

### Example 4

300 g of a perfume-free laundry detergent (trade name Persil perfume-free, commercially available from Henkel) are provided in a 2 1 sample container.

1.5 g of perfume particles on Portil A prepared according to Example 2 were added to the above described detergent formulation so that a perfume concentration of 0.1 wt.% based on the total weight of the final perfumed washing agent formulation was obtained and the mixture was shaken vigorously.

### Reference Example 1

A reference formulation was prepared as follows: 300 g of a perfume-free laundry detergent (trade name Persil perfume-free, commercially available from Henkel) were provided in a 21 sample container and perfume oil 'detergent perfume' was sprayed and dispersed in the formulation under agitation of the laundry detergent so that a final perfume concentration of 0.1 wt.% was obtained based on the total weight of the final formulation. The final mixture was vigorously shaken.

### Test 1 (detergent formulation)

The fragrance of the 3 perfumed laundry detergents of Example 3 and 4 and of Reference Example 1 was evaluated by 29 test persons. No significant differences of the formulations prepared by the addition of perfume particles to those obtained by the addition of liquid perfume were observed (see table 1 below).

### Test 2 (wet fabric)

The 3 final formulations of Example 3 and 4 and of Reference Example 1 were used as laundry detergents for towels and dishtowels.

The fragrance of the wet clothes was evaluated by 29 test persons. No significant differences of the formulations prepared by the addition of perfume particles to those obtained by the addition of liquid perfume were observed (see table 1 below).

### Test 3 (dry fabric)

The wet clothes of test 2 were dried on a leash. The fragrance of the dried fabric was evaluated by 29 test persons. No significant differences of the formulations prepared by the addition of perfume particles to those obtained by the addition of liquid perfume were observed (see table 1 below).

An overview of the fragrance properties of the final perfumed laundry detergents prepared according to Example 3 and 4 and to Reference Example 1 and of the textiles washed with the respective laundry detergents is provided in the following table 1. The test persons evaluated the pleasantness of the scent of the formulation fragrances (test 1) and of the textile fragrances (tests 2 and 3), respectively, on a scale from 1 (not acceptable) to 7 (excellent). The results represent an average over the marks from the 29 test persons:

| Detergent formulation | Test 1 Fragrance of the formulation | Test 2 Fragrance of the wet textile | Test 3 Fragrance of the dry textile |
|---|---|---|---|
| Example 3 | 4.1 | 4 | 3.1 |
| Example 4 | 3.9 | 4.4 | 3.4 |
| Reference Example 1 | 3.9 | 4.3 | 3.6 |

### Perfume particles for cleansing agents

Any known perfume composition suitable to be used in dishwashing agents, in the following called 'dishwashing perfume', can be employed in the preparation of the perfume particles described below.

### Example 5

16 g of a water-soluble sodium salt of the copolymer of maleic acid/acrylic acid with a weight molecular weight of 70,000 kg/kmol (trade name Sokalan CP 5, commercially available from BASF) were provided in a Petri dish. 4 g of the perfume oil 'dishwashing perfume' were sprayed and dispersed onto the polymer under agitation.

Subsequently, 4 wt.% of water-insoluble silica (Sipernat 50, commercially available from Degussa), based on the total weight of the polymer, the perfume and the silica, were added as powdering agent during mixing to yield flowable, non-sticky and non-adhering perfume particles.

### Example 6

16 g of the sodium salt of the copolymer of maleic acid/acrylic acid (trade name Sokalan CP 45, commercially available from BASF) were wetted with 4 g of perfume oil 'dishwashing perfume' by spraying the perfume onto the polymer during agitating. Subsequently, 3 wt.% of water-insoluble silica (trade name Sipernat 22 S, commercially available from Degussa) based on the total weight of the polymer, the perfume and silica, were added during mixing to yield free-flowable perfume particles.

### Example 7

4 g of the perfume oil 'dishwashing perfume' were sprayed onto 16 g of the sodium salt of the copolymerisate of maleic acid/olefin with a weight-average molecular weight of 12000 kg/kmol (trade name Sokalan CP 9, commercially available from BASF). Subsequently, 3 wt.% of water-insoluble silica (trade name Sipernat 22 S, commercially available from Degussa) based on the total weight of the polymer, the perfume and the silica, were added during mixing to yield free-flowable perfume particles.

### Example 8

32 g of water glass (trade name Portil A, commercially available from Cognis) were provided in a Petri dish. 8 g of the perfume oil 'dishwashing perfume' were sprayed onto the water glass during agitation.

Subsequently, 5 wt.% of water-insoluble silica (tradename Sipernat 22 S, commercially available from Degussa), based on the total weight of the water glass, the perfume and the silica, were added as powdering agent during mixing. Non-sticky, flowable perfume particles were obtained.

### Preparation of cleansing agents

The perfume particles produced as described above in examples 5 and 8 were mixed in a 5 1 Lödige-mixer with 2 kg of a non-perfumed dishwashing agent (Cognis specification ADD 01-05.987). The amount of the perfume particles was chosen such that a perfume concentration of 0.1 wt.% based on the total weight of the final perfumed dishwashing agent was obtained. A perfumed dishwashing agent with 0.1 wt.% perfume based on the total weight of the final formulation was prepared as comparative formulation by adding liquid perfume 'dishwashing perfume' to the non-perfumed dishwashing agent (ADD 01-05.987). The resulting formulations were tested for the fragrance of surfaces cleaned therewith and for their cleaning performance.

Following materials were tested in the dishwasher: glass, porcelain, steel cutlery, silver cutlery, plastics made based on melamine, polypropylene (PP) and styrene-acrylonitrile (SAN).

None of the above listed materials showed a remaining fragrance after cleaning in the dishwasher with the different formulations prepared as described above and no deposition of the cleaning formulation on their surfaces was observed. Furthermore, no unpleasant fragrance was noticed when opening the dishwasher.

The cleaning performance of the formulations comprising perfume particles on the basis of Sokalan CP5 (example 5) and Portil A (example 8) in comparison to the performance of the comparative formulation prepared by the addition of liquid perfume is represented in the following table 2:

| | Glass | Porcelain | Cutlery | Melamine | PP | SAN |
|---|---|---|---|---|---|---|
| Sokalan CP5 | + | ++ | 0 | 0 | + | + |
| Portil A | 0 | 0 | 0 | 0 | 0 | 0 |
| - significantly worse, - slightly worse, 0 comparable, + slightly better, ++ significantly better | | | | | | |

## Claims

1. Perfume particles comprising a perfume, a carrier material, which is water-soluble or dispersible in water, the carrier material being selected from builders and anti-redeposition agents, and a water-insoluble silica as powdering agent, which are obtainable by spraying the perfume onto the carrier material and subsequently adding the powdering agent during mixing, wherein the powdering agent is present in an amount in the range of 1-5 wt.% based on the total weight of the carrier material, the perfume and the powdering agent.

2. The perfume particles according to claim 1, wherein the perfume is present in an amount of 10-35 wt.% of the total weight of the carrier material, the perfume and the powdering agent.

3. The perfume particles according to claim 1 or 2, wherein the carrier material is selected from the group consisting of silicates; poly(meth)acrylic acid and salts thereof; cellulose; cellulose derivatives; starch; hydrolysates of starch; sucrose and polysaccharide acetate.

4. The perfume particles according to claim 3, wherein the carrier material is represented by water glass.

5. The perfume particles according to claim 3, wherein the carrier material is represented by a maleic acid/acrylic acid copolymer and its salts.

6. A process for preparing perfume particles as defined in any of the claims 1 to 5, which comprises spraying the perfume onto the carrier material supplied in the form of particles during agitation and subsequently adding the powdering agent during mixing.

7. Household products comprising perfume particles according to any one of claims 1 to 5.

8. Cosmetics or personal care products comprising perfume particles according to any one of claims 1 to 5.

9. Sanitary products comprising perfume particles according to any one of claims 1 to 5.

## Patentansprüche

1. Parfümteilchen, umfassend ein Parfüm, ein Trägermaterial, das wasserlöslich oder in Wasser dispergierbar ist, wobei das Trägermaterial ausgewählt ist aus Aufbaustoffen und Mitteln zur Verhinderung der erneuten Ausfällung, und ein wasserunlösliches Silika als pulverförmiges Mittel, die erhältlich sind durch Sprühen des Parfüms auf das Trägermaterial und anschließendes Zugeben des pulverförmigen Mittels während des Mischens, worin das pulverförmige Mittel in einer Menge im Bereich von 1-5 Gew.% vorhanden ist, bezogen auf das Gesamtgewicht des Trägermaterials, des Parfüms und des pulverförmigen Mittels.

2. Parfümteilchen nach Anspruch 1, worin das Parfüm in einer Menge von 10-35 Gew.% des Gesamtgewichtes des Trägermaterials, des Parfüms und des pulverförmigen Mittels vorhanden ist.

3. Parfümteilchen nach Anspruch 1 oder 2, worin das Trägermaterial ausgewählt ist aus der Gruppe bestehend aus Silikaten, Poly(meth)acrylsäure und Salzen davon, Cellulose, Cellulosederivaten, Stärke, Hydrolysaten von Stärke, Sucrose und Polysaccharidacetat.

4. Parfümteilchen nach Anspruch 3, worin das Trägermaterial durch Wasserglas dargestellt ist.

5. Parfümteilchen nach Anspruch 3, worin das Trägermaterial durch ein Maleinsäure/Acrylsäure-Copolymer und seine Salze dargestellt ist.

6. Verfahren zur Herstellung der Parfümteilchen wie in einem der Ansprüche 1 bis 5 definiert, umfassend das Sprühen des Parfüms auf das Trägermaterial, das in der Form von Teilchen während des Rührens zugeführt ist, und anschließende Zugabe des pulverförmigen Mittels während des Mischens.

7. Haushaltprodukte, umfassend Parfümteilchen nach einem der Ansprüche 1 bis 5.

8. Kosmetika oder Körperpflegeprodukte, umfassend Parfümteilchen nach einem der Ansprüche 1 bis 5.

9. Hygieneartikel, umfassend Parfümteilchen nach einem der Ansprüche 1 bis 5.

## Revendications

1. Particules de parfum comprenant un parfum, un matériau support qui est soluble ou peut être dispersé dans l'eau, le matériau support étant choisi parmi les adjuvents et agents d'antiredéposition, ainsi qu'une silice insoluble dans l'eau agissant en tant qu'agent de mises en poudre pouvant être obtenues en pulvérisant le parfum sur le matériau support, puis en ajoutant l'agent de mise en poudre au cours du mélange, l'agent de mise en poudre étant présent en une quantité dans la plage de 1 %-5 % en poids par rapport au poids total du matériau support du parfum et de l'agent de mise en poudre.

2. Particules parfumées selon la revendication 1 dans lesquelles le parfum est présent en une quantité de 10 %-35 % en poids du poids total du matériau support, du parfum et de l'agent de mise en poudre.

3. Particules parfumées selon les revendications 1 ou 2 dans lesquelles le matériau support est choisi dans le groupe constitué par des silicates, l'acide poly(méth)acrylique et ses sels, la cellulose, les dérivés de cellulose, l'amidon, les hydrolisats d'amidon, le sucrose et l'acétate de polysaccharose.

4. Particules parfumées selon la revendication 3 dans lesquelles le matériau support est constitué par du verre soluble.

5. Particules parfumées selon la revendication 3 dans lesquelles le matériau support est constitué par un copolymère acide maléique/acide acrylique ou ses sels.

6. Procédé de préparation de particules parfumées selon l'une quelconque des revendications 1 à 5, comprenant les étapes consistant à pulvériser le parfum sur le matériau support fourni sous la forme de particules, sous agitation, puis à ajouter l'agent de mise en poudre au cours du mélange.

7. Produits ménagers comprenant des particules parfumées selon l'une quelconque des revendications 1 à 5.

8. Produits de soins cosmétiques ou de personnes comprenant des particules parfumées selon l'une quelconque des revendications 1 à 5.

9. Produits sanitaires comprenant des particules parfumées selon l'une quelconque des revendications 1 à 5.
